# EUROPEAN PATENT APPLICATION

(11) **EP 4 736 669 A1**
(43) Date of publication of application: **06.05.2026**
(21) Application number: 24860081.9
(22) Date of filing: 13.06.2024
(51) Int. Cl.: A23L 33/105, A23L 33/125, A61K 8/9794, A61K 8/73, A61K 8/02, A61Q 19/00, A61Q 90/00

(54) **COMPOSITION INCLUDING NIPA PALM EXTRACT AND FUCOIDAN EXTRACT FOR REDUCING SKIN SWELLING**

(30) Priority: 25.08.2023 KR 20230112273
(71) Applicant: Kolmar Holdings Co., Ltd., Sejong 30004 (KR)
(72) Inventor: PARK, Byung Chul, Suwon-si Gyeonggi-do 16688 (KR); KIM, Seul Ki, Seoul 06798 (KR); KIM, You Ah, Seoul 06800 (KR); CHOI, Hyeong, Seoul 06800 (KR); YOON, Sang Hyun, Seoul 06638 (KR); MOON, Byoung Seok, Anyang-si Gyeonggi-do 14105 (KR)
(74) Representative: Nederlandsch Octrooibureau
(86) International application number: PCT/KR2024/008144
(87) International publication number: WO 2025/048157

(57) **Abstract**

The present invention relates to a composition including a nipa palm extract and a fucoidan extract for reducing skin swelling. Specifically, the composition has excellent effects in at least one or more of reducing swelling, improving hydration, enhancing elasticity, and increasing skin density.

## Description

### TECHNICAL FIELD

The present invention relates to a composition including a Nypa fruticans extract and a fucoidan extract for reducing skin swelling, and more particularly, to a composition having an excellent effect in alleviating swelling by including a Nypa fruticans extract and a fucoidan extract as active ingredients.

### BACKGROUND ART

Skin swelling occurs when excessive water or salt accumulates in the skin. This reduces skin elasticity and hydration, contributing to wrinkles and aging. Severe skin swelling may lead to skin cracking, loss of skin elasticity and radiance, and increased wrinkles.

Such skin swelling is known to be caused by a variety of factors, including dehydration, excessive salt intake, stress, increased free radicals, lack of sleep, allergic reactions, and hormonal imbalances.

A variety of products, including functional foods and cosmetics, are being released to reduce skin swelling, but most of these products lack significant effects or may cause skin irritation due to physical pressure or chemical ingredients.

Meanwhile, with the growing demand for products made from natural ingredients that are non-irritating to the human body and do not cause skin problems, extensive research is being conducted to discover and enhance the efficacy of natural ingredients. Furthermore, research is actively underway on mixtures of different natural extracts, utilizing the functions of various natural extracts. However, mixing one extract with another may lead to problems such as precipitation, formulation instability, and reduced user experience, and the desired effects are often not fully implemented.

Therefore, the present inventors have conducted ongoing research to develop a composition effective in reducing skin swelling using a combination of natural extracts, and as a result, discovered that by including a Nypa fruticans extract and a fucoidan extract as active ingredients, a composition may be produced that exhibits an excellent effect in at least one of alleviating skin swelling, improving hydration, enhancing elasticity, and increasing skin density, thereby completing the present invention.

### DETAILED DESCRIPTION OF THE INVENTION

### TECHNICAL PROBLEM

An objective of the present invention is to provide a composition including a Nypa fruticans extract and a fucoidan extract as active ingredients, thereby exhibiting an excellent effect in at least one of alleviating skin swelling, improving hydration, enhancing elasticity, and increasing skin density.

The technical problems of the present invention are not limited to above-mentioned technical problems, and other technical problems that are not mentioned will be readily understood by those skilled in the art from the description below.

### TECHNICAL SOLUTION

According to one embodiment of the present invention, a composition including a Nypa fruticans extract and a fucoidan extract for reducing skin swelling is provided.

### ADVANTAGEOUS EFFECTS

The composition according to the present invention has an excellent effect in at least one of alleviating skin swelling, improving skin hydration, enhancing skin elasticity, and increasing skin density.

### DESCRIPTION OF THE DRAWINGS

To help better understand the drawings cited in the detailed description of the present invention, a brief description of each drawing is provided.
FIG. 1 shows a diagram illustrating the antioxidant effects (radical scavenging activity) of examples and a comparative example.
FIG. 2 shows a diagram illustrating the results of reducing swelling in both cheeks, both calves, and both ankles in the placebo-control group, and the E-001 and E-002 administration groups before administration and after two weeks of administration.
FIG. 3 is a photographic representation of the results shown in FIG. 2.
FIG. 4 shows a diagram illustrating the results of improving skin moisture in both cheeks and the backs of both hands in the placebo-control group, and the E-001 and E-002 administration groups before administration and after two weeks of administration.
FIG. 5 shows a diagram illustrating the results of enhancing skin elasticity in both cheeks, the backs of both hands, and both calves in the placebo-control group, and the E-001 and E-002 administration groups before administration and after two weeks of administration.
FIG. 6 shows a diagram illustrating the results of increasing skin density on both cheeks, the backs of both hands, and both calves in the placebo-control group, E-001, and E-002 groups before administration and after two weeks of administration.
FIG. 7 is a photographic representation of the results shown in FIG. 6.

### BEST MODE

According to an embodiment of the present invention, a composition for reducing skin swelling, including a Nypa fruticans extract and a fucoidan extract, is provided.

In addition, the Nypa fruticans extract may be obtained by hot water extraction or ethanol extraction.

In addition, the fucoidan extract may be obtained by hot water extraction.

In addition, the Nypa fruticans extract and the fucoidan extract may be in dry powder form.

In addition, the composition may include the Nypa fruticans extract and the fucoidan extract in a weight ratio of 1:0.5 to 1:2.

In addition, the composition may include the Nypa fruticans extract and the fucoidan extract in a weight ratio of 1:1.

In addition, the composition may include 40% to 70% by weight of the Nypa fruticans extract and the fucoidan extract based on a total weight of the composition.

In addition, the composition may have an excellent effect in at least one of reducing swelling, improving hydration, enhancing elasticity, and increasing skin density.

In addition, a food composition including the composition is provided.

In addition, a cosmetic composition including the composition is provided.

Unless defined otherwise, all technical and scientific terms used herein have the same meaning as commonly understood by one of ordinary skill in the art to which the invention pertains. Generally, the nomenclature used herein and the experimental methods which will be described later are those well known and commonly employed in the art. Furthermore, when describing embodiments of the present invention, detailed descriptions of related known components or functions will be omitted if they are deemed to hinder understanding of the embodiments of the present invention. Furthermore, while embodiments of the present invention will be described below, the technical spirit of the present invention is not limited or restricted thereto, and can be modified and implemented in various ways by those skilled in the art.

When a part is herein said to "comprise" a certain component, unless specified otherwise, this means that it may further include other components, rather than exclude other components. The term "and/or" as used herein includes a combination of a plurality of related items or any one of a plurality of related items.

According to an embodiment of the present invention, a composition for reducing skin swelling, including a Nypa fruticans extract and a fucoidan extract, is provided.

The nipa palm(*Nypa fruticans*), the only species in the subfamily Nypa, a monotypic subfamily of the palm family, is distributed throughout South Asia, Southeast Asia, and Oceania. Nypa fruticans refers to a young shoot of the palm tree and is extremely high in polyphenols, which have antioxidant, blood circulation-promoting, anti-inflammatory, and cancer-preventing effects. Nypa fruticans is also rich in vitamin E and saponins, which help with cardiovascular disease, cataracts, and arthritis. Nypa fruticans is also known to have efficacy in constipation alleviation, detoxification, and hangover alleviation.

Fucoidan is a sulfated, high-molecular weight polysaccharide having a viscous structure found in trace amounts in brown algae such as wakame and kelp. Fucoidan is an ingredient that protects the algae from seawater currents and external stimuli, serving as a protective barrier against marine microorganisms.

In the present invention, the term "extract" refers to a substance obtained by extracting components from a natural product. For example, it is a broad concept that includes substances obtained by extracting solvent-soluble components from a natural product using water or an organic solvent, and substances obtained by extracting only specific components from a natural product. The term "extract" may encompass all extracts, fractions, dilutions, concentrates, or dried products thereof obtained in each step, such as extraction, fractionation, or purification.

In the present invention, extraction may be performed by utilizing any extraction method commonly used in the art, such as hot water extraction, ethanol extraction, immersion extraction, cold maceration extraction, reflux extraction, supercritical extraction, subcritical extraction, dissolution, compression, high-temperature extraction, high-pressure extraction, or ultrasonic extraction. Specifically, hot water extraction or ethanol extraction may be used, but the present invention is not limited thereto.

In an embodiment, the solvent used in the extract may be water, ethanol, or a mixture thereof, specifically purified water or ethanol. However, the present invention is not limited thereto, and various solvents applicable in the art may be used alone or in combination. For example, the extraction solvent may include water (or distilled water); lower alcohols having 1 to 4 carbon atoms, such as methanol, ethanol, propyl alcohol, and butyl alcohol; polyhydric alcohols, such as propanediol, butanediol, hexanediol, methylpropanediol, glycerin, butylene glycol, propylene glycol, pentylene glycol, and dipropylene glycol; and hydrocarbon solvents, such as methyl acetate, ethyl acetate, acetone, benzene, hexane, diethyl ether, and dichloromethane; or mixtures thereof.

In an embodiment, the Nypa fruticans extract may be obtained by hot water extraction or ethanol extraction.

In an embodiment, the fucoidan extract may be obtained by hot water extraction.

Hot water extraction uses hot water at temperatures between 60 and 140 °C to extract active ingredients. Because pure water is used as an extraction medium, hot water extraction has the advantage of avoiding problems arising from the toxicity of the organic solvent used during extraction.

In an embodiment, the extract may be in liquid form or in dried powder form. Various drying methods commonly used in the art, such as freeze drying, vacuum drying, hot air drying, and spray drying, may be applied.

In an embodiment, the extract may be prepared or processed through various methods, such as vacuum distillation or concentration.

The composition of the present invention includes both a Nypa fruticans extract and a fucoidan extract, and has an effect in at least one of reducing skin swelling, specifically, alleviating swelling, improving hydration, enhancing elasticity, and increasing skin density. Better effects can be obtained when the Nypa fruticans extract and the fucoidan extract are combined than when they are used alone.

The composition of the present invention may include the Nypa fruticans extract and the fucoidan extract in a weight ratio of 1:0.5 to 1:2. When the Nypa fruticans extract and the fucoidan extract are mixed in this ratio, the effects of alleviating swelling, improving hydration, enhancing elasticity, and increasing skin density can be enhanced.

In an embodiment, the Nypa fruticans extract and the fucoidan extract may be mixed in a weight ratio of 1:1. In this case, the effects of alleviating swelling, improving hydration, enhancing elasticity, and increasing skin density can be maximized. However, the present invention is not limited thereto.

In an embodiment, the Nypa fruticans extract and the fucoidan extract may be included in an amount of 40% to 70% by weight based on a total weight of the composition. When the Nypa fruticans extract and the fucoidan extract are included in the above-mentioned amount, an excellent skin swelling alleviation effect can be exhibited. However, the present invention is not limited thereto.

In an embodiment, the composition may exhibit an effect of at least one of alleviating swelling, improving hydration, enhancing elasticity, and increasing skin density. Specifically, the composition of the present invention can alleviate skin swelling by improving skin hydration, enhancing elasticity and increasing density, and suppressing factors that cause skin swelling.

In an embodiment, a food composition including the composition is provided.

The term "food" as used herein refers to a food manufactured and processed using raw materials or ingredients with beneficial functional properties for the human body. The term refers to a food with high medical or therapeutic effects, processed to effectively exhibit bioregulatory functions in addition to providing nutrition. The term may be used interchangeably with terms known in the art, such as functional foods. For example, the food may be manufactured as a health functional food, a health supplement, a special nutritional supplement, a functional beverage, and the like, or the composition according to the present invention may be added to natural foods, processed foods, and the like. The food may be manufactured as powder, granules, tablets, capsules, syrups, or beverages. There is no limitation on the form the food may take, and the food may encompass all foods in the conventional sense.

For example, the food may include beverages and various drinks, fruits and processed foods thereof (canned fruits, jams, etc.), fish, meat and processed foods thereof (ham, bacon, etc.), breads and noodles, cookies and snacks, dairy products (butter, cheese, etc.), and the like, and it may include all functional foods in the conventional sense. In addition, it may also include food used as animal feed.

The food composition according to the present invention may further include food additives and other suitable auxiliary ingredients commonly used in the art. For example, the composition may further contain flavoring agents, natural carbohydrates, sweeteners, vitamins, electrolytes, colorants, pectic acid, alginic acid, organic acids, protective colloid thickeners, pH adjusters, stabilizers, preservatives, glycerin, alcohols, carbonating agents, and the like.

For example, as a flavoring agent, natural flavoring agents including thaumatin, stevia extract, and the like, or synthetic flavoring agents including saccharin, aspartame, and the like may be used. As a natural carbohydrate, monosaccharides such as glucose and fructose, disaccharides including maltose and sucrose, polysaccharides including dextrin and cyclodextrin, and sugar alcohols including xylitol, sorbitol, erythritol, and the like may be used. As a sweetening agent, natural sweeteners such as thaumatin, stevia extract, or synthetic sweeteners such as saccharin, aspartame, and the like may be used. The food additives, other auxiliary components, and the like are preferably added and used in a trace amount as long as the purpose for which they are added may be achieved. The trace amount, when expressed numerically, may be in the range of 0.0005% to about 0.5% by weight based on a total weight of the food composition. Those skilled in the art may easily select the blending amount of the above-mentioned components within a range that does not impair the purpose and effect of the present invention.

In an embodiment, a cosmetic composition including the composition is provided. In the present invention, the term "skin" refers to tissue covering the body surface of an animal, and is a broad concept encompassing not only tissue covering the body surface of the face or body, but also the scalp.

In an embodiment, when the composition of the present invention is used as a cosmetic composition, it may be manufactured into various products effective in reducing swelling, improving skin hydration, enhancing skin elasticity, and increasing skin density.

In an embodiment, the composition of the present invention may be manufactured into functional cosmetics such as nourishing creams, essences, and ampoules, or basic cosmetics such as toners and lotions. Furthermore, it may be manufactured into soaps, cleaning agents, cleansing creams, and cleansing water, or into color cosmetics such as lipsticks, lip balms, mascara, blushers, shading products, highlighters, makeup bases, foundations, pacts, concealers, and skin covers. In addition, it may be manufactured as a product that adheres to the skin, such as a pack or hydrogel slimming patch, or as a product that is sprayed onto the skin, such as a mist or aerosol.

According to an embodiment, the cosmetic composition of the present invention may be provided in a formulation commonly manufactured in the art. For example, it may be provided in the form of a solution, an emulsion obtained by dispersing an oil phase in an aqueous phase, an emulsion obtained by dispersing an aqueous phase in an oil phase, a suspension, an emulsion, a solid, a gel, an oil, powder, a paste, or a foam aerosol.

According to an embodiment, other ingredients commonly used in the art, such as thickeners, pH adjusters, isotonic agents, surfactants, stabilizers, preservatives, antiseptics, UV absorbers, sterilizers, moisturizers, blockers, antioxidants, organic pigments, inorganic pigments, fragrances, vitamins, and the like may be further included, as long as the effects of the present invention are not impaired. Those skilled in the art may easily select the blending amounts of these ingredients, as long as they do not impair the purpose and effects of the present invention.

Hereinafter, the following examples and experimental examples are presented to further illustrate the present invention, but the present invention is not limited thereto.

### Preparation of Nypa fruticans hot water extract

A 10-fold volume of distilled water was added to each gram of Nypa fruticans, and extraction was performed with the resulting mixture under hot water at 95±2 °C for six hours. The solids from the extract obtained by the hot water extraction were removed, and the remaining solution was vacuum-concentrated. After concentration, the resulting concentrate was mixed with dextrin in a 7:3 ratio and spray-dried to obtain a Nypa fruticans hot water extract.

### Preparation of Nypa fruticans ethanol extract

A 10-fold volume of 50% ethanol was added to each gram of Nypa fruticans, and the extraction temperature was set at 70 °C. First extraction was performed in a solvent with a 10-fold volume of the sample volume (v/w) for four hours, and second extraction was performed in a solvent with a 10-fold volume of the sample volume (v/w) for two hours. After the extraction was complete, the solids were removed, and the remaining solution was vacuum-concentrated and then freeze-dried to obtain a Nypa fruticans ethanol extract.

### Preparation of fucoidan hot water extract

Dried seaweed sporophyll was extracted with purified water, and the extract was concentrated after solid-liquid separation to prepare a primary concentrate. After removing alginate from the concentrate, a secondary concentration was performed. Subsequently, polysaccharides were separated by adding ethanol and steeping, followed by purification of the precipitate. After purification, the precipitate was dissolved in water and freeze-dried to prepare a fucoidan hot water extract.

### Experimental Example 1. Antioxidant activity measurement (2,2-diphenyl-1-picrylhydrazyl (DPPH) radical scavenging assay)

Compositions including the above-prepared Nypa fruticans hot water extract, Nypa fruticans ethanol extract, or fucoidan hot water extract alone were used as Preparation Examples 1 to 3, while compositions mixed in the ratios shown in [Table 1] were used as Preparation Examples 4 to 9. Meanwhile, vitamin C was used as Comparative Example 1.

**[Table 1]**

| Classification | Components and mixing ratio |
|---|---|
| Comparative Example 1 | Vitamin C |
| Preparation Example 1 | Nypa fruticans hot water extract |
| Preparation Example 2 | Nypa fruticans ethanol extract |
| Preparation Example 3 | Fucoidan hot water extract |
| Preparation Example 4 | Nypa fruticans hot water extract + Fucoidan hot water extract, 2:1 mixture |
| Preparation Example 5 | Nypa fruticans hot water extract + Fucoidan hot water extract, 1:1 mixture |
| Preparation Example 6 | Nypa fruticans hot water extract + Fucoidan hot water extract, 2:2 mixture |
| Preparation Example 7 | Nypa fruticans ethanol extract + Fucoidan hot water extract, 2:1 mixture |
| Preparation Example 8 | Nypa fruticans ethanol extract + Fucoidan hot water extract, 1:1 mixture |
| Preparation Example 9 | Nypa fruticans ethanol extract + Fucoidan hot water extract, 1:2 mixture |

To measure the antioxidant activity of the preparation examples and the comparative example, a DPPH radical scavenging assay was performed. DPPH powder was dissolved in ethanol to prepare a 0.5 mM DPPH solution. 100 µL of each of the preparation examples and the comparative example was added to a 96-well plate. 100 µL of 99% ethanol was added to the non-DPPH reaction group, and 100 µL of 0.5 mM DPPH was added to the DPPH reaction group. The absorbance was measured at 540 nm.

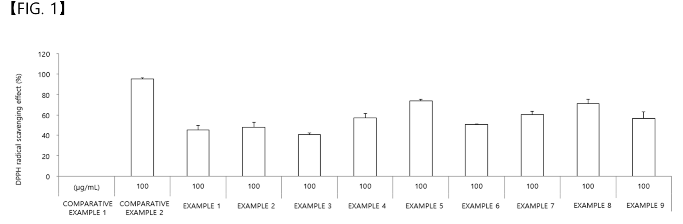

FIG. 1 shows a diagram illustrating the antioxidant activity (radical scavenging activity) of the preparation examples and the comparative example. As shown in FIG. 1, antioxidant activity was confirmed in all the preparation examples. In particular, Preparation Examples 4 to 9, in which the extracts were mixed, exhibited superior antioxidant activity compared to Preparation Examples 1 to 3, which contained either the Nypa fruticans extract or the fucoidan extract alone. Moreover, in the case of Preparation Examples 5 and 8, in which the Nypa fruticans extract and the fucoidan extract were mixed in a 1:1 ratio, the antioxidant effect was superior to that of the other preparation examples.

### Experimental Example 2. Skin swelling measurement

A clinical trial was conducted using Preparation Examples 5 and 8, which demonstrated the best antioxidant effects.

Adults aged 20 to 60 (61 subjects in the final efficacy evaluation, mean age: 43.541 years, 6 males, 55 females) were randomly divided into a placebo control group and an experimental group, and a skin swelling measurement test was conducted. Both the experimental group and placebo control group took 1000 mg tablets containing the ingredients listed in Table 2 twice daily (morning and evening) for two weeks.

The E-001 administration group was administered a composition containing a 1:1 mixture of the Nypa fruticans hot water extract and the fucoidan hot water extract (Preparation Example 5) . The E-002 administration group was administered a composition containing a 1:1 mixture of the Nypa fruticans ethanol extract and the fucoidan hot water extract (Preparation Example 8). The placebo control group took placebo tablets of the same shape as the experimental group. Unlike the Nypa fruticans ethanol extract, the Nypa fruticans hot water extract contained dextrin that was added during the spray-drying process. Therefore, the Nypa fruticans extract of Preparation Example 5 was included in an amount of 35.7143% by weight of a total composition weight, resulting in a 1:1 active ingredient mixing ratio, as in Preparation Example 8.

**[Table 2]**

| Ingredient Name | E-001 Administration Group | E-002 Administration Group | Placebo Control Group |
|---|---|---|---|
| Fucoidan extract | 25.0000% | 25.0000% | - |
| Nypa fruticans extract | 35.7143% | 25.0000% | - |
| Microcrystalline cellulose | 20.0357% | 18.7500% | 68.7500% |
| Aquamin F | 3.0000% | 5.0000% | 5.0000% |
| Dextrin | 5.0000% | 1.0000% | 15.0000% |
| Carboxymethylcellulose calcium | 4.0000% | 4.0000% | 4.0000% |
| Silicon dioxide | 1.8000% | 1.8000% | 1.8000% |
| Magnesium stearate | 1.5000% | 1.5000% | 1.5000% |
| Hydroxypropyl methylcellulose | 2.5000% | 2.5000% | 2.5000% |
| Glycerin fatty acid ester | 0.2500% | 0.2500% | 0.2500% |
| Titanium dioxide | 1.0000% | 1.0000% | 1.0000% |
| Coloring agent | 0.2000% | 0.2000% | 0.2000% |
| **Total** | **100.00000%** | **100.00000%** | **100.00000%** |

The degree of improvement in swelling in the subjects was measured before and after administration.

Specifically, skin swelling was measured using a 3D skin imaging device, Antera 3D CS (Miravex Ltd., Ireland). Images of the upper eyelids on both sides, the cheeks on both sides, and the backs of both hands were captured before and after administration for the placebo control group, E-001 administration group, and E-002 administration group. The images were then analyzed for skin volume (mm³) within a specific range (Elevation - Custom mode) . A decrease in the value indicates a greater swelling reduction effect.

Skin swelling of the calves, ankles, and dorsum of the feet was measured using the Vectra XT/Vectra H2 (Canfield Imaging Systems, USA), capable of measuring skin volume using 3D images. Images of the calves, ankles, and backs of the feet were captured before and after administration for the placebo control group, E-001 administration group, and E-002 administration group. The volume (cc) values were analyzed from the images. A decrease in the value indicates a greater swelling alleviation effect.

The measurement results and specific data are shown in Table 3 below.

**[Table 3]**

| Body part | | Change rate (%) | | |
|---|---|---|---|---|
| | | Placebo Control | E-001 | E-002 |
| Eyelid | Left | -6.301 | -11.433 | -13.558 |
| | Right | -3.873 | -10.593 | -14.011 |
| Cheek | Left | -1.235 | -14.275 | -13.694 |
| | Right | -1.225 | -14.346 | -13.617 |
| Back of hand | Left | 2.572 | -11.527 | -10.423 |
| | Right | 2.37 | -9.968 | -11.485 |
| Calf | Left | -0.61 | -9.178 | -10.397 |
| | Right | -0.855 | -9.897 | -9.591 |
| Ankle | Left | -2.899 | -17.008 | -16.105 |
| | Right | 0.118 | -15.074 | -13.764 |
| Dorsum of foot | Left | -1.759 | -19.317 | -13.091 |
| | Right | 0.142 | -20.088 | -13.559 |

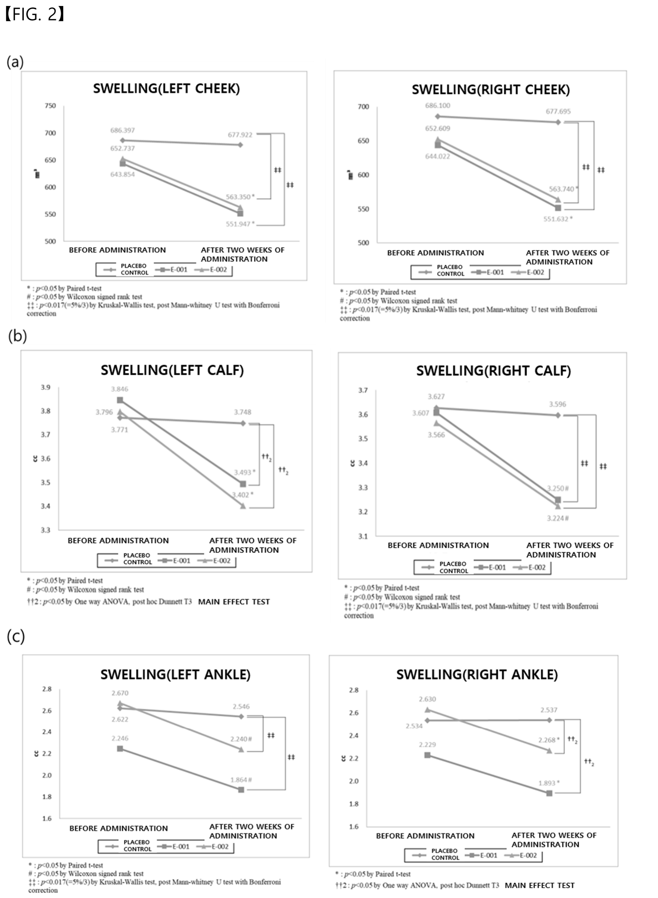

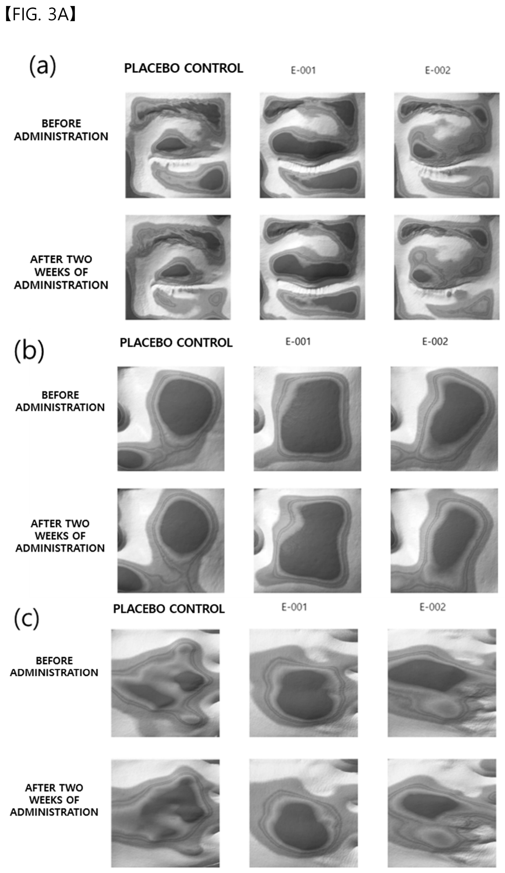

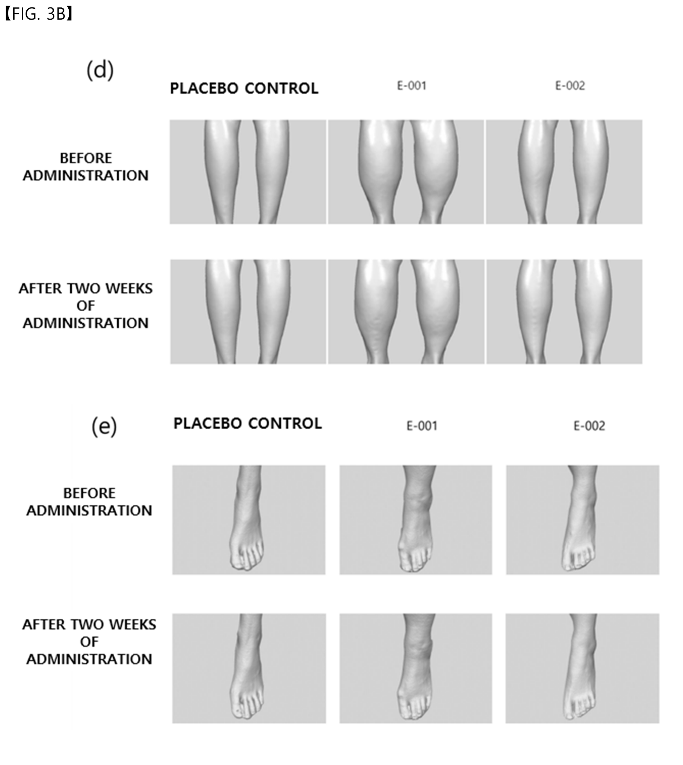

Based on the above-described data, FIG. 2 shows the results of reducing skin swelling of both cheeks, both calves, and both ankles before administration and after two weeks of administration in the placebo control group, E-001 administration group, and E-002 administration group in the form of a graph. In addition, in order to visually confirm the swelling reduction effect, images taken before and after administration are shown in FIG. 3. Specifically, in FIG. 3A, (a) is an image of the left eyelid before and after the administration, (b) is an image of the left cheek before and after the administration, (c) is an image of the left hand before and after the administration, and in FIG. 3B, (d) is an image of the calf before and after the administration, and (e) is an image of the ankle and the dorsum of the foot before and after the administration.

Referring to Table 3 and FIGS. 2 and 3, the placebo control group showed no statistically significant difference after two weeks of administration compared to before the administration, while the E-001 and E-002 administration groups showed a statistically significant decrease after two weeks of administration compared to before the administration. In other words, statistically significant differences were observed between the E-001 and E-002 administration groups and the placebo control group. Furthermore, the images taken showed no change in the placebo control group, while the E-001 and E-002 administration groups showed a significant reduction in swelling before and after the administration.

Therefore, it can be seen that the composition of the present invention is effective in reducing swelling across the entire skin, including the eyelids, cheeks, backs of the hands, calves, ankles, and dorsum of the feet.

### Experimental Example 3. Skin moisture measurement

The amount of moisture in skin was measured from the subjects of Experimental Example 2.

The amount of moisture in skin was measured using the MoistureMeter D Compact (Delfin Technologies Ltd., Finland) on the eyelids on both sides, cheeks on both sides, backs of both hands, calves on both sides, ankles on both sides, and dorsum of both feet in the placebo control group, E-001 administration group, and E-002 administration group before and after administration. The parameter was the tissue dielectric constant (TDC), expressed in %, and the measured value is proportional to the moisture content.

The detailed measurement results are presented in [Table 4] below.

**[Table 4]**

| Body part | | Change rate (%) | | |
|---|---|---|---|---|
| | | Placebo Control | E-001 | E-002 |
| Eyelid | Left | 0.209 | -1.015 | -1.997 |
| | Right | 0.211 | -0.785 | -1.964 |
| Cheek | Left | 0.097 | -1.491 | -1.961 |
| | Right | 0.000 | -1.493 | -1.897 |
| Back of hand | Left | 0.463 | -1.994 | -1.838 |
| | Right | 0.435 | -1.751 | -2.195 |
| Calf | Left | 0.564 | 1.258 | -3.705 |
| | Right | 1.515 | -1.442 | -2.894 |
| Ankle | Left | -0.920 | -1.212 | -7.117 |
| | Right | 1.073 | -1.548 | -4.136 |
| Dorsum of foot | Left | 0.194 | -2.186 | -4.034 |
| | Right | -0.797 | -1.260 | -3.840 |

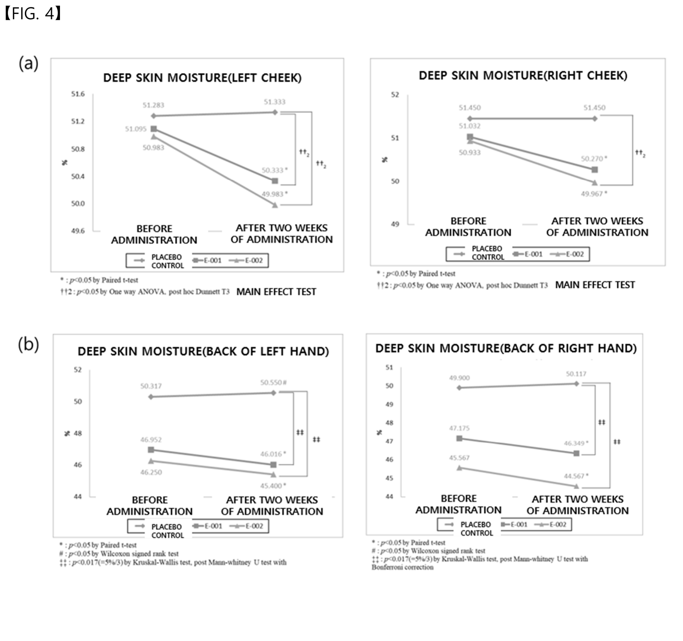

Based on the above-described data, FIG. 4 presents the results of skin moisture measurements on both cheeks and the backs of both hands for the placebo control group and the E-001 and E-002 administration groups before administration and after two weeks of administration.

Referring to Table 4 and FIG. 4, the placebo control group showed no statistically significant difference after two weeks of administration compared to before administration, but the E-001 and E-002 administration groups showed a statistically significant decrease after two weeks of administration compared to before administration. In other words, the E-001 and E-002 administration groups showed a statistically significant difference compared to the placebo control group.

Therefore, it can be seen that the composition of the present invention is effective in reducing swelling by reducing the skin moisture that causes swelling.

### Experimental Example 4. Skin elasticity measurement

Skin elasticity was measured for the subjects in Experimental Example 2.

Skin elasticity of the eyelids, cheeks, and backs of the hands was measured using a Cutometer MPA580 (Courage+ Khazaka electronic GmbH, Germany) . The measurements were taken on the eyelids on both sides, cheeks on both sides, and backs of both hands before and after administration in the placebo control group and the E-001 and E-002 administration groups. Measurements were performed three times, and the average value was used as the skin elasticity assessment data. The R2 value, which represents the skin's resilience, is a measure of overall elasticity. A value closer to 1 indicates greater elasticity, and the measured value is proportional to skin elasticity.

Skin elasticity of the calves, ankles, and dorsum of the feet was measured using a Ballistometer BLS780 (DIA STRON, UK). The measurements were taken on both calves, both ankles, and the backs of the feet in the placebo control group and the E-001 and E-002 administration groups before and after administration. Measurements were performed three times, and the average value was used as the skin elasticity assessment data. The measurement parameter was CoR, and the measured value is proportional to skin elasticity.

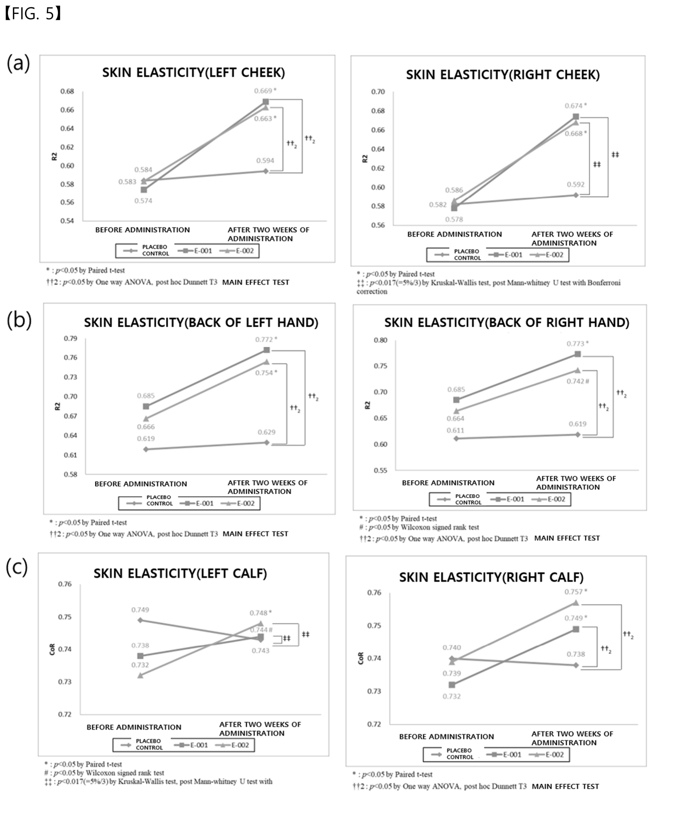

The detailed measurement results are shown in [Table 5] below. FIG. 5 is a graph illustrating the results of enhancing skin elasticity in both cheeks, the backs of both hands, and both calves in the placebo control group and the E-001 and E-002 administration groups before and after two weeks of administration.

**[Table 5]**

| Body part | | Change rate (%) | | |
|---|---|---|---|---|
| | | Placebo Control | E-001 | E-002 |
| Eyelid | Left | 1.835 | 11.786 | 13.781 |
| | Right | 1.441 | 11.935 | 13.904 |
| Cheek | Left | 1.712 | 16.551 | 13.722 |
| | Right | 1.718 | 16.609 | 13.993 |
| Back of hand | Left | 1.616 | 12.701 | 13.213 |
| | Right | 1.309 | 12.847 | 11.747 |
| Calf | Left | -0.801 | 0.813 | 2.186 |
| | Right | -0.270 | 2.322 | 2.436 |
| Ankle | Left | -0.603 | 1.881 | 3.395 |
| | Right | 0.304 | 1.082 | 1.967 |
| Dorsum of foot | Left | 0.374 | 3.208 | 4.545 |
| | Right | 1.657 | 4.029 | 4.114 |

Referring to Table 5 and FIG. 5, the placebo control group showed no statistically significant difference after two weeks of administration compared to before administration, whereas the E-001 and E-002 administration groups showed a statistically significant increase after two weeks of administration compared to before administration. In other words, statistically significant differences were observed between the E-001 and E-002 administration groups and the placebo control group.

Therefore, it can be seen that the composition of the present invention is effective in enhancing skin elasticity.

### Experimental Example 5. Skin density measurement

Skin density was measured on the subjects in Experimental Example 2.

Skin density was measured using a Skin Scanner DUB-USB (TPM taberna pro medicum, Germany). Scans were taken of the subjects' cheeks on both sides, the backs of both hands, both calves, and the dorsum of the feet before and after administration. The parameter was density, expressed in %, and the measured value is proportional to skin density.

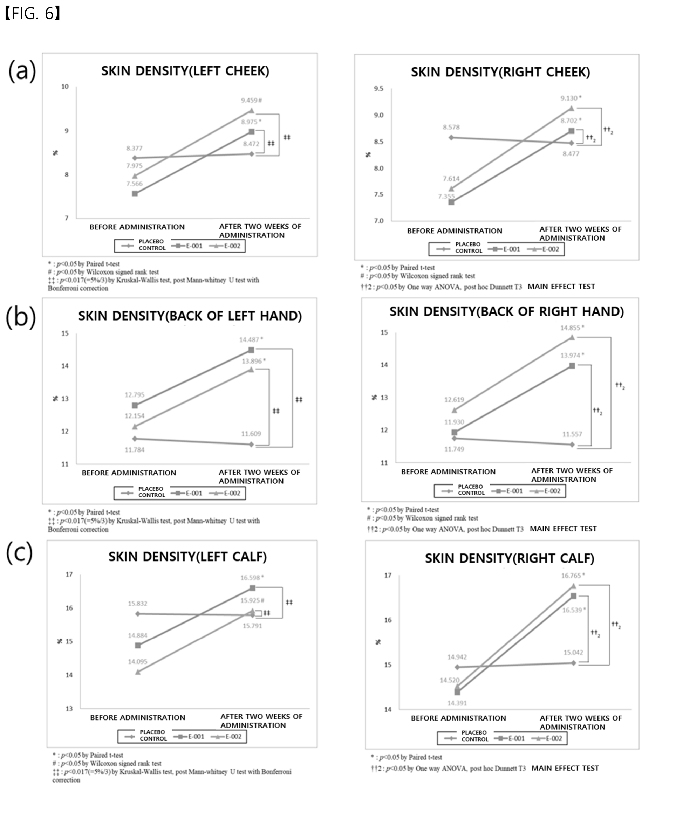

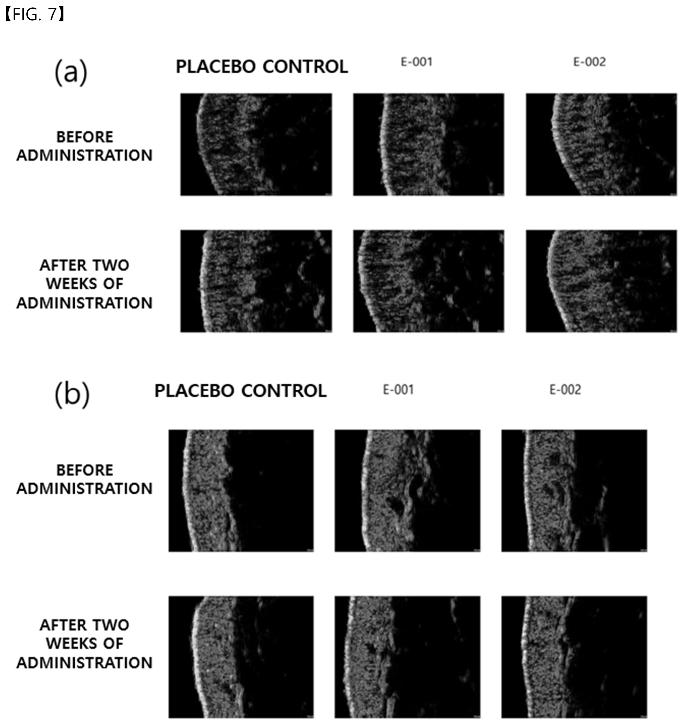

The detailed data from the measurement results are shown in [Table 6] below. FIG. 6 is a graph illustrating the increase in skin density in the cheeks on both sides, the backs of both hands, and both calves in the placebo control group and the E-001 and E-002 administration groups before and after two weeks of administration. FIG. 7 is a photographic representation of the measurement results. (a) is an image of the left cheek before and after administration, and (b) is an image of the right calf before and after administration.

**[Table 6]**

| Body part | | Change rate (%) | | |
|---|---|---|---|---|
| | | Placebo Control | E-001 | E-002 |
| Cheek | Left | 1.134 | 18.623 | 18.608 |
| | Right | -1.177 | 18.314 | 19.911 |
| Back of hand | Left | -1.485 | 13.224 | 14.333 |
| | Right | -1.634 | 17.133 | 17.719 |
| Calf | Left | -0.259 | 11.516 | 12.983 |
| | Right | 0.669 | 14.926 | 15.461 |
| Dorsum of foot | Left | -4.507 | 9.77 | 11.458 |
| | Right | 0.531 | 9.865 | 14.667 |

Referring to Table 6 and FIGS. 6 and 7, the placebo control group showed no statistically significant difference after two weeks of administration compared to before administration, whereas the E-001 and E-002 administration groups showed a statistically significant increase after two weeks of administration compared to before administration. In other words, compared to the placebo control group, the E-001 and E-002 administration groups showed a statistically significant difference after two weeks of administration. Furthermore, the images taken showed that the placebo control group showed no change, whereas the E-001 and E-002 administration groups showed a significant increase in skin density before and after administration.

Therefore, it can be seen that the composition of the present invention is effective in increasing skin density.

While specific aspects of the present invention have been described in detail above, it will be apparent to those skilled in the art that these specific descriptions merely represent preferred embodiments and are not intended to limit the scope of the present invention. Therefore, the substantial scope of the present invention is defined by the appended claims and their equivalents.

## Claims

1. A composition for reducing skin swelling, comprising a Nypa fruticans extract and a fucoidan extract.

2. The composition for reducing skin swelling of claim 1, wherein the Nypa fruticans extract is obtained by hot water extraction or ethanol extraction.

3. The composition for reducing skin swelling of claim 1, wherein the fucoidan extract is obtained by hot water extraction.

4. The composition for reducing skin swelling of claim 1, wherein the Nypa fruticans extract and the fucoidan extract are in dry powder form.

5. The composition for reducing skin swelling of claim 1, comprising the Nypa fruticans extract and the fucoidan extract in a weight ratio of 1:0.5 to 1:2.

6. The composition for reducing skin swelling of claim 1, comprising the Nypa fruticans extract and the fucoidan extract in a weight ratio of 1:1.

7. The composition for reducing skin swelling of claim 1, comprising 40% to 70% by weight of the Nypa fruticans extract and the fucoidan extract based on a total weight of the composition.

8. The composition for reducing skin swelling of claim 1, wherein the composition has an excellent effect in at least one of reducing swelling, improving hydration, enhancing elasticity, and increasing skin density.

9. A food composition comprising the composition according to any one of claims 1 to 8.

10. A cosmetic composition comprising the composition according to any one of claims 1 to 8.
